(19) Europäisches Patentamt
European Patent Office
Office européen des brevets

(11) Veröffentlichungsnummer : **0 356 694 B1**

(12) # EUROPÄISCHE PATENTSCHRIFT

(45) Veröffentlichungstag der Patentschrift : **01.04.92 Patentblatt 92/14**

(51) Int. Cl.$^5$ : **C07D 307/91,** A01N 43/12

(21) Anmeldenummer : **89113501.4**

(22) Anmeldetag : **22.07.89**

(54) **Dihydrodibenzofuranderivate und diese Verbindungen enthaltende Fungizide.**

(30) Priorität : **29.07.88 DE 3825840**

(43) Veröffentlichungstag der Anmeldung : **07.03.90 Patentblatt 90/10**

(45) Bekanntmachung des Hinweises auf die Patenterteilung : **01.04.92 Patentblatt 92/14**

(84) Benannte Vertragsstaaten : **AT BE CH DE ES FR GB GR IT LI NL SE**

(56) Entgegenhaltungen :
**EP-A- 0 057 362**
**EP-A- 0 177 222**
**DE-A- 2 060 205**

(73) Patentinhaber : **BASF Aktiengesellschaft**
**Carl-Bosch-Strasse 38**
**W-6700 Ludwigshafen (DE)**

(72) Erfinder : **Speakman, John-Bryan, Dr.**
**U 4,2**
**W-6800 Mannheim 1 (DE)**
Erfinder : **Karl, Rudolf, Dr.**
**Muehlweg 22**
**W-6703 Limburgerhof (DE)**
Erfinder : **Lorenz, Gisela, Dr.**
**Erlenweg 13**
**W-6730 Neustadt (DE)**
Erfinder : **Ammermann, Eberhard, Dr.**
**Sachsenstrasse 3**
**W-6700 Ludwigshafen (DE)**
Erfinder : **Wuerzer, Bruno, Dr.**
**Ruedigerstrasse 13**
**W-6701 Otterstadt (DE)**
Erfinder : **Meyer, Norbert, Dr.**
**Dossenheimer Weg 22**
**W-6802 Ladenburg (DE)**
Erfinder : **Ditrich, Klaus, Dr.**
**Paray-le-Monial-Strasse 12**
**W-6702 Bad Duerkheim (DE)**



Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft substituierte Benzofuranderivate, Verfahren zu ihrer Herstellung sowie Fungizide, die diese Verbindungen als Wirkstoffe enthalten.

Es ist bekannt, daß Usninsäure

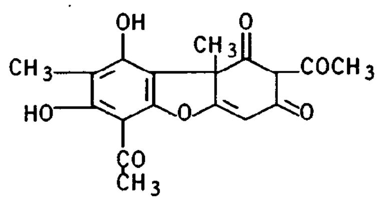

(2,6-Diacetyl-7,9-dihydroxy-8,9b-dimethyl-1,3(2H,9bH)-dibenzofuran-dion) antibakteriell wirksam ist (Römpp, Chemie Lexikon, 1966, Seiten 6820, 6821).

Es wurde nun gefunden, daß neue substituierte Benzofuranderivate der Formel I

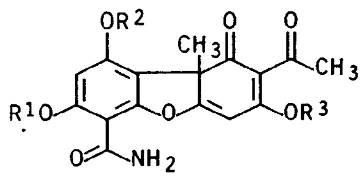

(I)

in der $R^1$, $R^2$, $R^3$ gleich oder verschieden sind und Wasserstoff, Alkali- oder Erdalkali $C_1$-$C_4$-Alkyl, $C_2$-$C_4$-Alkanoyl, halogensubstituiertes $C_2$-Alkanoyl, gegebenenfalls durch Halogen oder $C_1$-$C_4$-Alkyl substituiertes $C_7$-$C_9$-Phenylalkyl, gegebenenfalls durch Halogen oder $C_1$-$C_4$-Alkyl substituiertes Benzoyl oder einen Rest $R^4R^5R^6Si$ bedeuten, wobei $R^4$, $R^5$ und $R^6$ gleich oder verschieden sind und $C_1$-$C_6$-Alkyl bedeuten, eine fungizide Wirkung haben, die besser als die der Usninsäure ist.

$R^1$, $R^2$, $R^3$ in Formel I kann ein Alkali- oder Erdalkalimetall, z.B. Na, Mg, Ca oder einen $C_1$-$C_4$-Alkylrest, beispielsweise Methyl, Ethyl, n-Butyl, einen $C_2$-$C_4$-Alkanoylrest, beispielsweise Acetyl, Propionyl, Butyryl, einen halogen-substituierten Alkanoylrest, z.B. Trifluoracetyl, einen gegebenenfalls durch Halogen oder $C_1$-$C_4$-Alkyl substituierten $C_7$-$C_9$-Phenylalkyl- oder Benzoylrest, beispielsweise Benzyl, Phenylethyl, p-Chlor-benzyl, m-Chlor-benzyl, p-Methyl-benzyl oder einen Rest der Formel $R^4R^5R^6Si$, in dem $R^4$, $R^5$ und $R^6$ gleich oder verschieden sind und $C_1$-$C_4$-Alkyl, beispielsweise Methyl, Ethyl, n-Butyl, bedeuten.

Man erhält z.B. die Derivate der Formel I, wobei mindestens einer der drei Reste $R^1$ bis $R^3$ $C_1$-$C_4$-Alkyl oder gegebenenfalls durch Halogen oder $C_1$-$C_4$-Alkyl substituiertes $C_7$-$C_9$-Phenylalkyl bedeuten, durch Umsetzung von Verbindungen der Formel I, in der mindestens ein Rest $R^1$, $R^2$, $R^3$ Wasserstoff bedeutet, mit einem Alkylierungsmittel der Formel

$$R^7\text{-}X \qquad (II)$$

in der $R^7$ die obengenannten Bedeutungen für $R^1$, $R^2$, $R^3$ außer Wasserstoff hat und X für Halogen oder $OSO_2R^7$ steht, in Gegenwart eines inerten Lösungsmittels und gegebenenfalls in Gegenwart einer Base. Die Reaktion verläuft z.B. bei einer Temperatur im Bereich von 20 bis 100°C, vorzugsweise 40 bis 80°C.

Derivate der Formel I, wobei $R^1$ bis $R^3$ gleich oder verschieden sind und mindestens einer dieser drei Reste $C_2$-$C_4$-Alkanoyl, halogensubstituiertes $C_2$-Alkanoyl oder gegegenenfalls durch Halogen oder $C_1$-$C_4$-Alkyl substituiertes Benzoyl bedeutet, lassen sich z.B. durch Umsetzung von Verbindungen der Formel I, in der mindestens ein Rest $R^1$, $R^2$, $R^3$ Wasserstoff bedeutet, mit einem Anhydrid der Formel

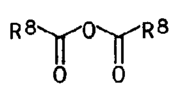

(III)

in der $R^8$ $C_1$-$C_3$-Alkyl, z.B. Methyl, Ethyl, Propyl, halogensubstituiertes $C_1$-Alkyl, z.B. Trifluormethyl oder gegebenenfalls durch Halogen oder $C_1$-$C_4$-Alkyl substituiertes Phenyl, Z.B. Chlorphenyl, Tolyl, Methoxyphenyl bedeuten, in Gegenwart eines inerten Lösungsmittels bei einer Temperatur zwischen 80 und 120°C oder mit einem säurechlorid der Formel

$R^8$-CO-Cl (IV)

in der $R^8$ die oben genannten Bedeutungen hat, gegebenenfalls in Gegenwart einer Base bei einer Temperatur zwischen 20 und 120°C, vorzugsweise zwischen 60 und 80°C, herstellen.

Die silylierten Derivate der Formel I, in denen $R^1$, $R^2$, $R^3$ gleich oder verschieden sind und mindestens einer dieser drei Reste den Rest der Formel $R^4R^5R^6Si$ bedeuten, können z.B. durch Umsetzung von Verbindungen der Formel I, in der mindestens ein Rest $R^1$, $R^2$, $R^3$ Wasserstoff bedeutet, mit einem Chlorsilan der Formel

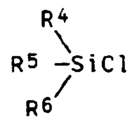 (V)

in der $R^4$, $R^5$ und $R^6$ einen $C_1$-$C_6$-Alkylrest bedeutet, in Gegenwart eines inerten Lösungsmittels und einer Base bei einer Temperatur zwischen 20 und 70°C oder mit einem Silylderivat der Formel $R^9CONHSiR^4R^5R^6$, in der $R^9$ Methyl oder Trifluormethyl bedeutet, und $R^4$, $R^5$, $R^6$ die oben genannten Bedeutung haben, oder mit einem Silylderivat der Formel

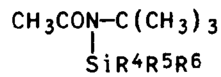 (VI)

bei einer Temperatur zwischen 0 und 50°C, vorzugsweise 30 und 40°C, erhalten werden.

Geeignete Silylderivate des Acetamids, des Trifluoracetamids oder des N-t-Butylacetamids sind beispielsweise die Trimethylsilyl-, Triethylsilyl- oder n-Butyldimethylsilylderivate.

Als Lösungsmittel kommen für die oben genannten Verfahren z.B. Halogenkohlenwasserstoffe, insbesondere Chlorkohlenwasserstoffe, z.B. Tetrachlorethylen, 1,1,2,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol; Ether, z.B. Ethylpropylether, Methyltert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, β,β'-Dichlordiethylether; Nitrokohlenwasserstoffe, wie Nitromethan, Nitroethan, Nitrobenzol, o-, m-, p-Chloronitrobenzol, o-Nitrotoluol; Nitrile, wie Acetonitril, Butyronitril, Isobutyronitril, Benzonitril, m-Chlorbenzonitril; aliphatische, cycloaliphatische oder aromatische Kohlenwasserstoffe, z.B. Heptan, Pinan, Nonan, o-, m-, p-Cymol, Benzinfraktionen innerhalb eines Siedepunktintervalls von 70 bis 190°C, Cyclohexan, Methylcyclohexan, Dekalin, Petrolether, Hexan, Liqroin, 2,4,4-Trimethylpentan, 2,2,3-Trimethylpentan, 2,3,3-Trimethylpentan, Octan, Toluol, o-, m-, p-Xylol, Tetralin; Ester, z.B. Ethylacetat, Acetessigester, Isobutylacetat; Amide, z.B. Formamid, Methylformamid, Dimethylformamid; Ketone, z.B. Aceton, Methylethylketon, Akohole, wie Methanol, Ethanol, Isopropanol, Sulfoxide, wie Dimethylsulfoxid, Heteroaromaten, wie Pyridin, α-, β-, γ-Picolin, Pyrimidin und entsprechende Gemische in Betracht. Zweckmäßig verwendet man das Lösungsmittel in einer Menge von 100 bis 2000 Gew.%, vorzugsweise von 200 bis 700 Gew.%, bezogen auf den Ausgangsstoff II, III, IV, V oder VI.

Als Basen für die Umsetzung von Verbindungen der Formel I, in der mindestens ein Rest $R^1$ bis $R^3$ Wasserstoff bedeutet, mit den Verbindungen der Formeln II, IV oder V kommen tertiäre Amine, Erdalkaliverbindungen, Ammoniumverbindungen und Alkaliverbindungen sowie entsprechende Gemische in Betracht. Auch Zinkverbindungen können verwendet werden. Beispiele hierfür sind Kaliumhydroxid, Natriumhydroxid, Kaliumcarbonat, Natriumcarbonat, Lithiumhydroxid, Lithiumcarbonat, Natriumhydrogencarbonat, Kaliumhydrogencarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumhydrogencarbonat, Magnesiumacetat, Zinkhydroxid, Zinkoxid, Zinkcarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert.-butylamin, Tribenzlamin, Tricyclohexylamin, Triamylamin, Diisopropylethylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidon, N-Ethypyrrolidon, N-Methylpiperidin, N-Ethypiperidin, N-Methylpyrrolidon, N-Ethylpyrrolidon, N-Methylimidazol, N-Ethylimidazol,

N-Methylhexamethylenimin, N-Ethylhexamethylenimin, Pyridin, Chinolin, α-Picolin, β-Picolin, γ-Picolin, Isochinolin, Pyrimidin, Acridin, N,N,N',N'-Tetramethylethylendiamin, N,N,N'N'-Tetraethylethylendiamin, Chinoxalin, Chinazolin, N-Propyldiisopropyl-amin, N,N'-Dimethylcyclohexylamin, 2,6-Lutidin, 2,4-Luditidn, Triflurylamin, Triethylendiamin.

Außer den vorgenannten Basen eignen sich z.B. Natriumpropionat, Natriumbutyrat, Natriumisobutyrat, Kaliumformiat, Kaliumacetat, Kaliumpropionat, Kaliumbutyrat, Kaliumisobutyrat, Natriummethylat, Natriumethylat, Natriumpropylat, Natriumisopropylat, Natriumbutylat, Natriumisobutylat, Natrium-sec-butylat, Natrium-tert.-butylat, Natriumethylenglykolat, Natriumpropylen-(1,2-)-glykolat, Natriumpropylen-(1,3)-glykolat, Natriumdiethylenglykolat, Natriumtriethylenglykolat, Natriumdipropylen-(1,2)-glykolat, Kaliummethylat, Kaliumethylat, Kalium-n-propylat, Kaliumisopropylat, Kalium-n-butylat, Kalium-iso-butylat, Kalium-sec-butylat, Kalium-tert.-butylat, Kaliummethylenglykolat, Kaliumpropylen-(1,2)-glykolat, Kaliumpropylen-(1,3)-glykolat, Kaliumdiethylenglykolat, Kaliumtriethylenglykolat, Kaliumdipropylen-(1,2)-glykolat.

Sämtliche Verfahren können kontinuierlich oder diskontinuierlich, drucklos oder unter Druck, durchgeführt werden; der Einfachheit halber wird Atmosphärendruck bevorzugt.

Beispiele

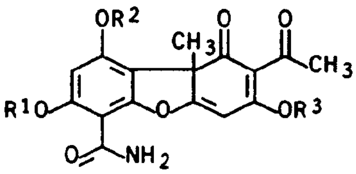

| Nr. | $R^1$ | $R^2$ | $R^3$ | Fp. |
|---|---|---|---|---|
| 1 | H | H | H | 180-182°C (Zers.) |
| 2 | $CH_3$ | $CH_3$ | H | |
| 3 | $CH_3CO$ | $CH_3CO$ | H | |
| 4 | $Si(CH_3)_3$ | $Si(CH_3)_3$ | H | |
| 1a | Na-Salz von Nr. 1 | | | |
| 1b | Mg-Salz von Nr. 1 | | | |
| 1c | Ca-Salz von Nr. 1 | | | |
| 8 | H | H | $CH_3$ | |
| 9 | H | H | $Si(CH_3)_3$ | |
| 10 | H | H | $COCH_3$ | |
| 11 | H | H | $COCF_3$ | |
| 12 | $CH_3$ | $CH_3$ | $CH_3$ | |
| 13 | $COCH_3$ | $COCH_3$ | $COCH_3$ | |
| 14 | $Si(CH_3)_3$ | $Si(CH_3)_3$ | $Si(CH_3)_3$ | |

Die Verbindung Nr. 1 2-Acetyl-3,7,9-trihydroxy-6-carbamido-9b-methyl-1(1H,9bH)-dibenzofuran-on kann z.B. durch Kultivierung des Pilzes DSM 4431 und Abtrennung aus dem Kulturfiltrat hergestellt werden.

Der Pilz DSM 4431 wurde aus einer Rasenprobe (Speyer, BRD) isoliert. Eine lebensfähige Kultur dieses Mikroorganismus wurde bei der Deutschen Sammlung für Mikroorganismen, Mascheroder Weg 1b, 3300 Braunschweig, hinterlegt und deren permanenter Sammlung einverleibt. Der Mikroorganismus ist der Öffentlichkeit bei dieser Hinterlegungsstelle unter seiner Hinterlegungsnummer DSM 4431 frei zugänglich.

Die Verbindung Nr. 1 wird, allgemein gesprochen, dadurch hergestellt, daß man den Pilz DSM 4431 unter aeroben Bedingungen in einem flüssigen Medium, enthaltend assimilierbare Quellen an Kohlenstoff, Stickstoff und anorganischen Anionen und Kationen, kultiviert, bis sich eine wesentlich Menge der Verbindung Nr. 1 in dem Medium gebildet hat und nachfolgend diese Verbindung daraus gewinnt.

Die Verbindung Nr. 1 wird beispielsweise hergestellt, indem man unter aeroben Bedingungen ein flüssiges

Medium fermentiert, das assimilierbare Quellen an Kohlenstoff, Stickstoff und anorganischen Anionen und Kationen enthält, wobei das Medium mit einer lebensfähigen Kultur des Pilzes DSM 4431 oder einer die Verbindung Nr. 1 produzierenden Mutante desselben beimpft wurde, den Organismus bei steriler Belüftung und unter Rühren während eines Zeitraumes von 50 bis 150 Stunden bei einer Temperatur von 20 bis 30°C und pH 5,5 bis 8,0 kultiviert, das Kulturfiltrat abtrennt und die Verbindung daraus extrahiert.

Die Extraktion der Verbindung Nr. 1 kann beispielsweise in der Weise erfolgen, daß man sie aus dem Kulturfiltrat mit einem mit Wasser nicht mischbaren Lösungsmittel oder nach Gefriertrocknung des Kulturfiltrates mit einem Lösemittel aus diesem extrahiert.

Die Reinigung des Extraktes kann beispielsweise durch chromatographische Verfahren erfolgen.

Für die Anwendung als Fungizid ist nicht nur die Verbindung Nr. 1 als solche, sondern auch die Fermentationsbrühe oder Gesamtmaische des Mikroorgaismus DMS 4431 geeignet.

Man kann auch den aus der Fermentationsbrühe oder Gesamtmaische des Mikroorganismus DSM 4431 gewonnenen Extrakt verwenden.

Die Verbindung Nr. 1 entsteht im Verlauf der Kultivierung des Pilzes DSM 4431 unter kontrollierten Bedingungen.

Allgemeine Fermentationsbedingungen

Die Kultivierung des Pilzes DSM 4431 kann in einer breiten Vielfalt flüssiger Kulturmedien durchgeführt werden. Geeignete Nährmedien umfassen eine assimilierbare Quelle von Kohlenstoff, wie Dextrin, Saccharose, Melasse, Glycerin etc.; eine assimilierbare Quelle an Stickstoff, wie Protein, Proteinhydrolysat, Polypeptide, Aminosäure, Maisquellwasser etc.; sowie an organische Anionen und Kationen, wie Kalium, Natrium, Ammonium, Calcium, Sulfat, Carbonat, Phosphat, Chlorid etc. Spurenelemente wie Bor, Molybdän, Kupfer, Mangan, Zink, Eisen etc. werden in Form von Verunreinigungen der anderen Bestandteile der Medien oder als definierte Lösungen den Medien zugeführt. Die Belüftung in Flaschen und Tanks wird durchgeführt, indem man sterile Luft durch das Närmedium hindurchleitet oder auf die Oberfläche des Nährmediums aufpreßt. Eine weitere Bewegung in den Tanks wird durch einen mechanischen Rührer gewährleistet. Falls erforderlich, kann ein Antischaum-Mittel, wie Siliconöl, zugesetzt werden.

Beispiel a

Inokulum-Herstellung

Geeignete Medien, wie sie zur Anzucht des Inokulums verwendet werden, sind Malz-Extrakt (2 %) oder Zuckerrüben-Melasse (2 %).

aa) Für die Fermentation in kleinem Maßstab

Die Nährmedien werden sterilisiert. Der gleichmäßig mit der keimfreien Kultur des Pilzes DSM 4431 bewachsene Agar (2 % Malz-Extrakt) einer Glasschale (9 cm ∅) wird in einem sterilisierten Mixer unter Zusatz von 200 ml sterilisiertem Wasser (entionisiert) homogenisiert und anschließend werden jeweils 40 ml dieses Homogenisates als Inokulum für die Kultur des Pilzes in Glaskolben (jeweils 500 ml Nährmedium) verwendet.

ab) Für die Fermentation in größerem Maßstab

Die Nährmedien werden sterilisiert. Der gleichmäßig mit der keimfreien Kultur des Pilzes DSM 4431 bewachsene Agar (2 % Malz-Extrakt) von 5 Glasschalen (9 cm ∅) wird in einem sterilisierten Mixer unter Zusatz von 400 ml sterilisiertem Wasser (entionisiert) homogenisiert und dann jeweils die Hälfte dieses Homogenisates als Inokulum in 1 l Glaskolben (mit jeweils 500 ml Nährmedium) überführt. Die Medien werden anschließend 24 bis 30 h bei 25°C auf einem Rotationschüttler (130 bis 150 U/min) geschüttelt. Dieses Inokulum wird anschließend verwendet, um 15 l eines sterilen Zuckerrüben-Melasse-Mediums (2 %) zu beimpfen.

Beispiel b

Fermentation in kleinem Maßstab

Zur Bereitung der Fermentationsmedien wurde Zuckerrüben-Melasse (2 %) verwendet. Nach dem Beimpfen der sterilisierten Medien (jeweils 500 ml Nährmedium pro Kolben) mit dem nach Beispiel aa hergestellten Inokulum werden diese anschließend 5 bis 6 Tage bei 25°C auf einem Rotationsschüttler (130 bis 150 U/min) geschüttelt und dann die Maische geerntet.

Beispiel c

Fermentation in größerem Maßstab

Zur Bereitung der Fermentationsmedien wurde Zuckerrüben-Melasse (2 %) verwendet. Nach dem Beimpfen der sterilisierten Medien (jeweils 15 l Nährmedium) mit dem nach Beispiel ab hergestellten Inokulum wird die Fermentation bei 25°C, wobei ein steriler Luftstrom von 1,5 bis 2 l/min aufrechterhalten und unter Rühren mit einem Blattrührer, der mit 150 U/min betrieben wird, durchgeführt. Die Fermentation wird 5 bis 8 Tage betrieben und anschließend die Maische geerntet.

Analog diesem Beispiel werden Fermentationen im 50 l-, 100- bzw. 2000 l- Maßstab durchgeführt, wobei die Menge an Inokulum und der sterile Luftstrom den geänderten Volumina an Nährmedium entsprechend angepaßt werden.

Beispiel d

Isolierung von Verbindung Nr. 1

Ein Gesamtvolumen von ca. 80 Liter Maische, hergestellt gemäß Beispiel b oder c, wird filtriert und der Mycelkuchen verworfen. Das so erhaltene Kulturfiltrat wird in einem kontinuierlichen Extraktionsverfahren mit Dichlormethan extrahiert und aus diesem Extrakt anschließend am Rotationsverdampfer unter Vakuum das Lösungsmittel entfernt. Der Rückstand wird in einem geringen Volumen einer Mischung aus Dichlormethan:Dioxan:Methanol 65:35:5 aufgenommen und auf eine vorher mit der mobilen Phase equilibrierten Säule aufgetragen und dann druckunterstützt chromatographiert.

Bedingungen:

| | |
|---|---|
| Säule: | innerer Durchmesser 5,4 cm<br>Füllhöhe 60,0 cm |
| stationäre Phase: | Siliciumdioxid 25-40μm<br>(Merck LichroPrep Si 60) |
| mobile Phase: | Dichlormethan:Dioxan:Methanol = 60:35:5 |
| Flußrate: | 45 ml/min |
| Druck: | 1 bar |

Detektion des Eluates mittels Photometer: $\lambda$ = 254 bzw. 260 nm.

Die den Hauptanteil an der biologisch wirksamen Substanz enthaltende Fraktion wird am Rotationsverdampfer unter Vakuum zur Trockene eingeengt und anschließend in wenig Methanol aufgenommen. Durch Zugabe von Wasser läßt sich die wirksame Verbindung auskristallisieren und durch mehrmaliges Umkristallisieren erhält man eine sehr reine Substanz. Die so erhaltene verbindung liegt teilweise als Ca-Mg-Salz vor, wobei offensichtlich die Anteile an Ca- bzw. Mg in Abhängigkeit von den Kulturbedingungen für den Mikroorganismus mehr oder minder schwanken können. Die Verbindung Nr. 1 wird dadurch erhalten, daß man entsprechende Mengen der aus den Kulturfiltraten isolierten Ausgangsverbindung in 2 %iger Schwefelsäure unter Zusatz von aceton (wegen der geringen Löslichkeit der Verbindung in Wasser) löst, 2 h bei Raumtemperatur (20°C) rührt und anschließend mit $CH_2Cl_2$ extrahiert. Nach Einengen der $CH_2Cl_2$-Phase am Rotationsverdampfer unter Vakuum wird der Rückstand in Methanol aufgenommen und unter Zusatz von Wasser auskristallisiert. Durch mehrmalige Umkristallisierungen wird die reine Verbindung Nr. 1 erhalten.

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Uncinula necator an Reben,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspension, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 1 mit 10 Gew.- Teilen N.Methyl-$\alpha$-pyrrolidon und erhält eine Lösung, die zur Anwendung in Form kleinster Tropfen geeignet ist.

II. 20 Gew.-Teile der Verbindung Nr. 1 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 1 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 1 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 1 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 1 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 1 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung es fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:
Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
O,O-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,

4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol,
N-(3-Chlor-2,6-dinitro-4-trifluormethyl-phenyl)-5-trifluormethyl-3-chlor-2-aminopyridin,
1-((bis-(4-Fluorphenyl)-methylsilyl)-methyl)-1H-1,2,4-triazol.

Anwendungsbeispiele

Als Vergleichswirkstoffe wurden die bekannten Usninsäuren (rechsdrehend oder linksdrehend) benutzt.

Anwendungsbeispiel 1

Wirksamkeit gegen Botrytis cinerea an Paprika

Paprikasämlinge der Sorte "Neusiedler Ideal Elite" wurden, nachdem sich 4 bis 5 Blätter gut entwickelt hatten, mit wäßrigen Suspensionen, die 80 % (Gew.%) Wirkstoff und 20 % Emulgiermittel in der Trockensubstanz

enthielten, tropfnaß gespritzt. Nach dem Antrocknen des Spritzbelages wurden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

| Wirkstoff | Befall der Blätter in Prozent nach Applikation von 0,05 %iger wäßriger Wirktoffaufbereitung |
|---|---|
| 1 | 3 |
| Bekanntes Vergleichsmittel | |
| + Usninsäure | 40 |
| - Usninsäure | 40 |
| Unbehandelt: | 100 |

Anwendungsbeispiel 2

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20 - 22°C und 70 % relativer Luftfeuchtigkeit für weitere 5 Tage kultiviert. Dann wurde das Ausmaß der Symptomentwicklung ermittelt.

| Wirkstoff | Befall der Blätter in Prozent nach Applikation von 0,05 %iger wäßriger Wirktoffaufbereitung |
|---|---|
| 1 | 10 |
| Bekanntes Vergleichsmittel | |
| + Usninsäure | 20 |
| - Usninsäure | 20 |
| Unbehandelt: | 100 |

## Patentansprüche

1. Substituierte Benzofuranderivate der Formel I

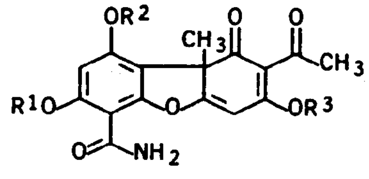

(I)

in der $R^1$, $R^2$, $R^3$ gleich oder verschieden sind und Wasserstoff, Alkali- oder Erdalkalimetall, $C_1$-$C_4$-Alkyl, gegebenenfalls durch Halogen oder $C_1$-$C_4$-Alkyl substituiertes $C_7$-$C_9$ phenylalkyl, $C_2$-$C_4$-Alkanoyl, halogensubstituiertes $C_2$-Alkanoyl, gegebenenfalls durch Halogen oder $C_1$-$C_4$-Alkyl substituiertes Benzoyl oder einen Rest

$R^4R^5R^6Si$ bedeuten, wobei $R^4$, $R^5$ und $R^6$ gleich oder verschieden sind und $C_1$-$C_4$-Alkyl bedeuten.

2. Substituierte Benzofuranderivate der Formel I gemäß Anspruch 1, in der $R^1$, $R^2$ und $R^3$ gleich oder verschieden sind und Wasserstoff oder Alkali- oder Erdalkalimetall bedeuten.

3. Verbindung der Formel I gemäß Anspruch 1, dadurch gekennzeichnet, daß $R^1$, $R^2$ und $R^3$ Wasserstoff bedeuten.

4. Verfahren zur Herstellung von Benzofuranderivaten der Formel I gemäß Anspruch 1, wobei $R^1$, $R^2$, $R^3$ gleich oder verschieden sind und mindestens einen diesen dri Rute $C_1$-$C_4$-Alkyl oder gegebenenfalls durch Halogen oder $C_1$-$C_4$-Alkyl substituiertes $C_7$-$C_9$-Phenylalkyl bedeutet, dadurch gekennzeichnet, daß man eine Verbindung der Formel I, in der mindestens einer der Reste $R^1$, $R^2$, $R^3$ Wasserstoff bedeutet, mit einem Alkylierungsmittel der Formel

$$R^7\text{-}X \qquad (II)$$

in der $R^7$ die obengenannten Bedeutungen für $R^1$, $R^2$, $R^3$ außer Wasserstoff hat und X für Halogen oder $OSO_2R^7$ steht, in Gegenwart eines Lösungsmittels und gegebenenfalls in Gegenwart einer Base umsetzt.

5. Verfahren zur Herstellung von Benzofuranderivaten der Formel I gemäß Anspruch 1, wobei $R^1$, $R^2$, $R^3$ gleich oder verschieden sind und mindestens einen dieser drei Rute $C_2$-$C_4$-Alkanoyl, halogensubstituiertes $C_2$-Alkanoyl sowie gegebenenfalls durch Halogen oder $C_1$-$C_4$-Alkyl substituiertes Benzoyl bedeutet, dadurch gekennzeichnet, daß man eine Verbindung der Formel I, in der mindestens einer der Reste $R^1$, $R^2$, $R^3$ Wasserstoff bedeutet, mit einem Anhydrid der Formel

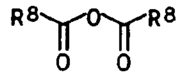

(III)

in der $R^8$ $C_1$-$C_3$-Alkyl, halogensubstituiertes $C_1$-Alkyl oder gegebenenfalls durch Halogen oder $C_1$-$C_4$-Alkyl substituiertes Phenyl bedeutet, in Gegenwart eines inerten Lösungsmittels oder mit einem Säurechlorid der Formel

$$R^8\text{-CO-Cl} \qquad (IV)$$

in der $R^8$ die oben genannten Bedeutungen hat, gegebenenfalls in Gegenwart einer Base umsetzt.

6. Verfahren zur Herstellung von Benzofuranderivaten der Formel I gemäß Anspruch 1, wobei $R^1$, $R^2$, $R^3$ gleich oder verschieden sind und mindestens einer diesen drei Rute den Rest $R^4R^5R^6Si$ bedeutet, dadurch gekennzeichnet, daß man eine Verbindung der Formel I, in der mindestens einer der Reste $R^1$, $R^2$, $R^3$ Wasserstoff bedeutet, mit einem Chlorsilan der Formel

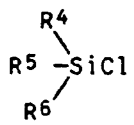

(V)

in der $R^4$, $R^5$ und $R^6$ $C_1$-$C_6$-Alkyl bedeuten, in Gegenwart einer Base oder mit einem Silylderivat der Formel $R^9CONHSiR^4R^5R^6$, in der $R^9$ Methyl oder Trifluormethyl bedeutet, und $R^4$, $R^5$, $R^6$ die oben genannten Bedeutungen haben, oder mit einem Silylderivat der Formel

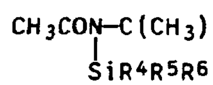

umsetzt.

7. Verfahren zur Herstellung von Benzofuranderivaten der Formel I gemäß Anspruch 1, wobei $R^1$, $R^2$, $R^3$ Wasserstoff bedeuten, dadurch gekennzeichnet, daß man den Pilz DSM 4431 oder ein diese Benzofuranderivate produzierende Mutante desselben in einem flüssigen Medium, enthaltend assimilierbare Quellen von Kohlenstoff, Stickstoff und anorganische Anionen und Kationen unter aeroben Bedingungen fermentiert, bis sich eine wesentlich Menge an dem Benzofuranderivat in dem Medium gebildet hat und das Benzofuranderivat aus dem Medium gewinnt.

8. Verwendung einer fungizid wirksamen Menge der Fermentationsbrühe oder der Gesamtmaische des Mikroorganismus DSM 4431 zur Bekämpfung von Pilzen, dadurch gekennzeichnet, daß man die Pilze oder die vor Pilzbefall zu schützenden Saatgüter, Pflanzen, Materialien oder den Boden mit der Fermentationsbrühe

oder Gesamtmaische behandelt.

9. Fungizid, enthaltend einen inerten Zusatzstoff und eine fungizid wirksame Menge eines substituierten Benzofuranderivats der Formel I

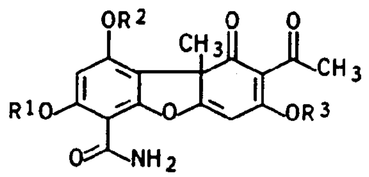

(I)

in der $R^1$, $R^2$, $R^3$ gleich oder verschieden sind und Wasserstoff, Alkali- oder Erdalkalimetall, $C_1$-$C_4$-Alkyl, gegebenenfalls durch Halogen oder $C_1$-$C_4$-Alkyl substituiertes $C_7$-$C_9$-Phenylalkyl, $C_2$-$C_4$-Alkanoyl, halogensubstituiertes $C_2$-Alkanoyl, gegebenenfalls durch Halogen oder $C_1$-$C_4$-Alkyl substituiertes Benzoyl oder einen Rest $R^4R^5R^6Si$ bedeuten, wobei $R^4$, $R^5$ und $R^6$ gleich oder verschieden sind und $C_1$-$C_4$-Alkyl bedeuten.

10. Verfahren zur Bekämpfung von Pilzen, dadurch gekennzeichnet, daß man diese oder die vor Pilzbefall zu schützenden Saatgüter, Pflanzen, Materialien oder den Boden mit einer fungizid wirksamen Menge eines substituierten Benzofuranderivats der Formel I

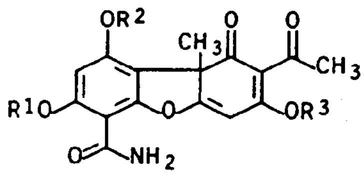

(I)

in der $R^1$, $R^2$, $R^3$ gleich oder verschieden sind und Wasserstoff, Alkali- oder Erdalkalimetall, $C_1$-$C_4$-Alkyl, gegebenenfalls durch Halogen oder $C_1$-$C_4$-Alkyl substituiertes $C_7$-$C_9$-Phenylalkyl, $C_2$-$C_4$-Alkanoyl, halogensubstituiertes $C_2$-Alkanoyl, gegebenenfalls durch Halogen oder $C_1$-$C_4$-Alkyl substituiertes Benzoyl oder einen Rest $R^4R^5R^6Si$ bedeuten, wobei $R^4$, $R^5$ und $R^6$ gleich oder verschieden sind und $C_1$-$C_4$-Alkyl bedeuten, behandelt.

## Claims

1. A substituted benzofuran derivative of the formula I

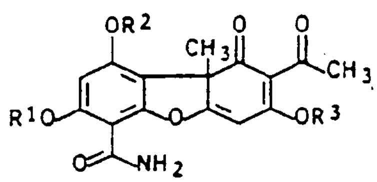

(I)

where $R^1$, $R^2$ and $R^3$ are identical or different and are each hydrogen, an alkali or alkaline earth metal, $C_1$-$C_4$-alkyl, unsubstituted or halogen- or $C_1$-$C_4$-alkyl-substituted $C_7$-$C_9$-phenylalkyl, $C_2$-$C_4$-alkanoyl, halogen-substituted $C_2$-alkanoyl, unsubstituted or halogen- or $C_1$-$C_4$-alkyl-substituted benzoyl or a radical $R^4R^5R^6Si$ in which $R^4$, $R^5$ and $R^6$ are identical or different and are each $C_1$-$C_4$-alkyl.

2. A substituted benzofuran derivative of the formula I as claimed in claim 1, where $R^1$, $R^2$ and $R^3$ are identical or different and are each hydrogen or an alkali metal or alkaline earth metal.

3. A compound of the formula I as claimed in claim 1, wherein $R^1$, $R^2$ and $R^3$ are each hydrogen.

4. A process for the preparation of a benzofuran derivative of the formula I as claimed in claim 1, where $R^1$, $R^2$ and $R^3$ are identical or different and one or more of these three radicals $C_1$-$C_4$-alkyl or unsubstituted or halogen- or $C_1$-$C_4$-alkyl-substituted $C_7$-$C_9$-phenylalkyl, wherein a compound of the formula I, where one or more of the radicals $R^1$, $R^2$ and $R^3$ are hydrogen, is reacted with an alkylating agent of the formula

$$R^7\text{-X} \qquad \text{(II)}$$

where $R^7$ has the abovementioned meanings for $R^1$, $R^2$ and $R^3$, with the exception of hydrogen, and X is halogen or $OSO_2R^7$, in the presence of a solvent and in the presence or absence of a base.

5. A process for the preparation of a benzofuran derivative of the formula I as claimed in claim 1, where $R^1$, $R^2$ and $R^3$ are identical or different and one or more of these three radicals is $C_2$-$C_4$-alkanoyl, halogen-substituted $C_2$-alkanoyl or unsubstituted or halogen- or $C_1$-$C_4$-alkyl-substituted benzoyl, wherein a compound of the formula I, where one or more of the radicals $R^1$, $R^2$ and $R^3$ is hydrogen, is reacted with an anhydride of the formula

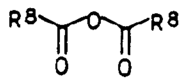

(III)

where $R^8$ is $C_1$-$C_3$-alkyl, halogen-substituted $C_1$-alkyl or unsubstituted or halogen- or $C_1$-$C_4$-alkyl-substituted phenyl, in the presence of an inert solvent, or with an acyl chloride of the formula

$R^7$-CO-Cl (IV)

where $R^8$ has the abovementioned meanings, in the presence or absence of a base.

6. A process for the preparation of a benzofuran derivative of the formula I as claimed in claim 1, where $R^1$, $R^2$ and $R^3$ are identical or different and one or more of these three radicals is the radical $R^4R^5R^6Si$, wherein a compound of the formula I, where at one or more of the radicals $R^1$, $R^2$ and $R^3$ is hydrogen, is reacted with a chlorosilane of the formula

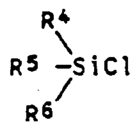

(V)

where $R^4$, $R^5$ and $R^6$ are each $C_1$-$C_6$-alkyl, in the presence of a base, or with a silyl derivative of the formula $R^9CONHSiR^4R^5R^6$, where $R^9$ is methyl or trifluoromethyl and $R^4$, $R^5$ and $R^6$ have the abovementioned meanings, or with a silyl derivative of the formula

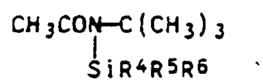

7. A process for the preparation of a benzofuran derivative of the formula I as claimed in claim 1, where $R^1$, $R^2$ and $R^3$ are each hydrogen, wherein the fungus DSM 4431, or a mutant thereof producing said benzofuran derivative, is fermented under aerobic conditions in a liquid medium containing assimilable sources of carbon, nitrogen and inorganic anions and cations, until a significant amount of the benzofuran derivative has formed in the medium, and the benzofuran derivative is isolated from the medium.

8. The use of a fungicidally effective amount of the fermentation broth or total slurry of the microorganism DSM 4431 for controlling fungi, wherein the fungi, or the seeds, plants, materials or the soil to be protected against fungus attack are treated with the fermentation broth or the total slurry.

9. A fungicide containing an inert additive and a fungicidally effective amount of a substituted benzofuran derivative of the formula I

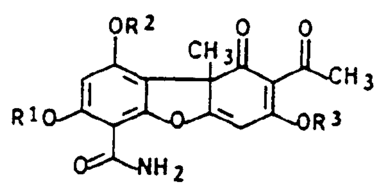

(I)

where $R^1$, $R^2$ and $R^3$ are identical or different and are each hydrogen, an alkali or alkaline earth metal, $C_1$-$C_4$-alkyl, unsubstituted or halogen- or $C_1$-$C_4$-alkyl-substituted $C_7$-$C_9$-phenylalkyl, $C_2$-$C_4$-alkanoyl, halogen-substituted $C_2$-alkanoyl, unsubstituted or halogen- or $C_1$-$C_4$-alkyl-substituted benzoyl or a radical $R^4R^5R^6Si$ in which $R^4$, $R^5$ and $R^6$ are identical or different and are each $C_1$-$C_4$-alkyl.

10. A process for controlling fungi, wherein the fungi or the seeds, plants, materials or soil to be protected against fungus attack are treated with a fungicidally effective amount of a substituted benzofuran derivative of the formula I

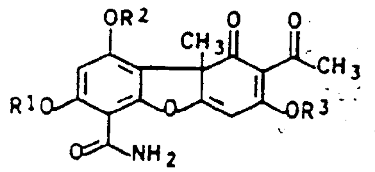

(I)

where $R^1$, $R^2$ and $R^3$ are identical or different and are each hydrogen, an alkali or alkaline earth metal, $C_1$-$C_4$-alkyl, unsubstituted or halogen- or $C_1$-$C_4$-alkyl-substituted $C_7$-$C_9$-phenylalkyl, $C_2$-$C_4$-alkanoyl, halogen-substituted $C_2$-alkanoyl, unsubstituted or halogen- or $C_1$-$C_4$-alkyl-substituted benzoyl or a radical $R^4R^5R^6Si$ in which $R^4$, $R^5$ and $R^6$ are identical or different and are each $C_1$-$C_4$-alkyl.

## Revendications

1. Dérivés de benzofuranne substitués, de formule I

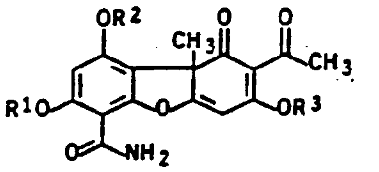

(I)

dans laquelle $R^1$, $R^2$, $R^3$ sont identiques ou différents et représentent hydrogène, métal alcalin ou alcalino-terreux, alkyle en C 1-C 4, phénylalkyle en C 7-C 9, éventuellement substitué par halogène ou alkyle en C 1-C 4, alkanoyle en C 2-C 4, alkanoyle en C 2 substitué par halogène, benzoyle, éventuellement substitué par halogène ou alkyle en C 1-C 4, ou un reste $R^4$ $R^5$ $R^6$ Si où $R^4$, $R^5$ et $R^6$ sont identiques ou différents et représentent alkyle en C 1-C 4.

2. Dérivés de benzofuranne substitués, de formule I selon la revendication 1, dans laquelle $R^1$, $R^2$ et $R^3$ sont identiques ou différents et représentent hydrogène ou métal alcalin ou alcalino-terreux.

3. Composé de formule I, selon la revendication 1, caractérisé par le fait que $R^1$, $R^2$ et $R^3$ représentent hydrogène.

4. Procédé de préparation de dérivés de benzofuranne de formule I, selon la revendication 1, $R^1$ , $R^2$, $R^3$ étant identiques ou différents et un de ces trois restes au moins représentent alkyle en C 1-C 4 ou phénylalkyle en C 7-C 9, éventuellement substitué par halogène ou alkyle en C 1-C 4, caractérisé par la fait que l'on fait réagir, en présence d'un solvant et éventuellement en présence d'une base, un composé de formule I, dans laquelle au moins un des restes $R^1$, $R^2$, $R^3$ représente hydrogène, avec un agent d'alkylation de formule

$$R^7 - X \qquad (II)$$

dans laquelle $R^7$ a les significations données plus haut pour $R^1$, $R^2$, $R^3$, excepté hydrogène, et X est mis pour halogène ou $OSO_2R^7$.

5. Procédé de préparation de dérivés de benzofuranne de formule I, selon la revendication 1, $R^{\&}$, $R^2$, $R^3$ étant identiques ou différents et un de ces trois restes au moins représentant alkanoyle en C 2-C 4, alkanoyle en C 2 substitué par halogène, ainsi que benzoyle éventuellement substitué par halogène ou alkyle en C 1-C 4, caractérisé par le fait que l'on fait réagir un composé de formule I, dans laquelle un des restes $R^1$, $R^2$, $R^3$ au moins représente hydrogène, avec un anhydride de formule

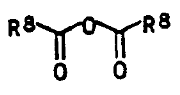

(III)

dans laquelle $R^8$ représente alkyle en C 1-C 3, alkyle en C 1 substitué par halogène ou phényle éventuellement substitué par halogène ou alkyle en C 1-C 4, en présence d'un solvant inerte, ou avec un chlorure d'acide de formule

$$R^8\text{-CO-C}\ell \qquad (IV)$$

dans laquelle $R^8$ a les significations données plus haut, éventuellement en présence d'une base.

6. Procédé de préparation de dérivés de benzofuranne de formule I, selon la revendication 1, dans laquelle $R^1$, $R^2$, $R^3$ sont identiques ou différents, un de ces trois restes au moins représentant un reste $R^4$, $R^5$, $R^6$ Si,

caractérisé par le fait que l'on fait réagir un composé de formule I, dans laquelle au moins un des restes $R^1$, $R^2$, $R^3$ représente hydrogène, avec un chlorosilane de formule

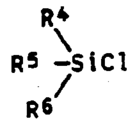

(V)

dans laquelle $R^4$, $R^5$ et $R^6$ représentent alkyle en C 1-C 6, en présence d'une base, ou avec un dérivé de silyle de formule $R^9CONHSiR^4R^5R^6$, dans laquelle $R^9$ représente méthyle ou trifluorométhyle, $R^4$, $R^5$, $R^6$ ayant les significations données plus haut, ou avec un dérivé de silyle de formule

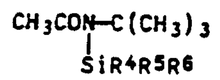

7. Procédé de préparation de dérivés de benzofuranne de formule I, selon la revendication 1, dans laquelle $R^1$, $R^2$, $R^3$ représentent hydrogène, caractérisé par le fait que l'on fermente, dans des conditions aérobies, le champignon DSM 4431 ou un mutant de celui-ci produisant des dérivés de benzofuranne, dans une milieu liquide contenant des sources assimilables de carbone, d'azote et d'anions et cations inorganiques, jusqu'à ce que se soit formée dans la milieu liquide une quantité importante du dérivé de benzofuranne dans le milieu liquide, et on isole le dérivé de benzofuranne dudit milieu.

8. Utilisation d'une quantité efficace, du point de vue fongicide, du bouillon de fermentation ou de la totalité de la trempe du micro-organisme DM 4431 pour la lutte contre les champignons, caractérisé par le fait que l'on traite les champignons ou les semences, plantes, matériaux ou le sol à protéger contre l'attaque par les champignons avec le bouillon de fermentation ou la totalité de la trempe.

9. Fongicide contenant un additif inerte et une quantité efficace du point de vue fongicide d'un dérivé de benzofuranne substitué, de formule I

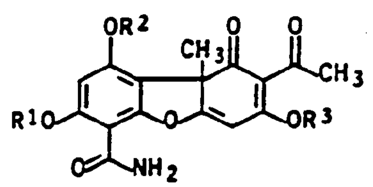

(I)

dans laquelle $R^1$, $R^2$, $R^3$ sont identiques ou différents et représentent hydrogène, métal alcalin ou alcalino-terreux, alkyle en C 1-C 4, phénylalkyle en C 7-C 9, éventuellement substitué par halogène ou alkyle en C 1-C 4, alkanoyle en C 2-C 4, alkanoyle en C 2, substitué par halogène, benzoyle, éventuellement substitué par halogène ou alkyle en C 1-C 4, ou un reste $R^4R^5R^6Si$, étant identiques ou différents et représentant alkyle en C 1-C 4.

10. Procédé de lutte contre les champignons, caractérisé par le fait que l'on traite les champignons ou les semences, plantes, matériaux ou le sol à protéger contre l'attaque par les champignons, avec une quantité efficace, du point de vue fongicide, d'un dérivé de benzofuranne substitué, de formule I

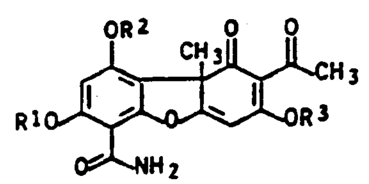

(I)

dans laquelle $R^1$, $R^2$, $R^3$ sont identiques ou différentes et représentent hydrogène, métal alcalin ou alcalino-terreux, alkyle en C 1-C 4, phénylalkyle en C 7-C 9, éventuellement substitué par halogène ou alkyle en C 1-C 4, alkanoyle en C 2-C 4, alkanoyle en C 2 substitué par halogène, benzoyle, éventuellement substitué par halogène ou alkyle en C 1-C 4, ou un reste $R^4$ $R^5$ $R^6Si$ où $R^4$, $R^5$ et $R^6$ sont identiques ou différents et représentent alkyle en C 1-C 4.